# EUROPÄISCHE PATENTSCHRIFT

(11) **EP 0 537 489 B1**
(45) Veröffentlichungstag und Bekanntmachung des Hinweises auf die Patenterteilung: **08.12.1999**
(21) Anmeldenummer: 92115878.8
(22) Anmeldetag: 17.09.1992
(51) Int. Cl.: C07K 16/00, C07K 14/435, C12P 21/08, C12N 5/20, C12N 15/12, G01N 33/574, G01N 33/577, A61K 39/395

(54) **Monoklonale Antikörper gegen tumorassoziierte Antigene, Verfahren zu ihrer Herstellung und ihre Verwendung**
Monoclonal antibodies against tumor-associated antigens, method for their preparation and their use
Anticorps monoclonaux contre des antigènes associés aux tumeurs, procédé de préparation et utilisation

(30) Priorität: 11.10.1991 DE 4133791
(43) Veröffentlichungstag der Anmeldung: 21.04.1993
(73) Patentinhaber: Dade Behring Marburg GmbH, 35001 Marburg (DE)
(72) Erfinder: Bosslet, Klaus, W-3550 Marburg (DE); Seemann, Gerhard, W-3550 Marburg-Elnhausen (DE); Pfleiderer, Peter, W-3550 Marburg (DE)

(56) Entgegenhaltungen:
- EP-A- 0 256 654
- EP-A- 0 376 746
- EP-A- 0 388 914
- EP-A- 0 397 419
- EP-A- 0 420 394
- EP-A- 0 443 599

## Beschreibung

Die vorliegende Erfindung betrifft monoklonale Antikörper gegen ein tumorassoziiertes Antigen, das vornehmlich von Tumoren aus der Gruppe der Mamma-, Ovarial-, Prostata- sowie Adenokarzinomen der Lunge stammt, die zusätzlich mit dem "polymorphic epithelial mucin (PEM)" reagieren, ihre Herstellung und Verwendung zur Diagnose und Therapie.

Durch die Hybridomatechnologie wurde die Möglichkeit geschaffen, spezifische monoklonale Antikörper (MAK) auch gegen nicht gereinigte Antigene herzustellen. Diese Tatsache ermöglichte die Identifikation einer Vielzahl von tumorassoziierten Antigenen (TAA), die auf bestimmten humanen Tumoren, aber auch auf Normalgeweben des Menschen vorkommen. Solche TAA sind z. B. das CEA (carcinoembryonic antigen), das N-CAM (neural cell adhesion molecule) und das PEM (polymorphic epithelial mucin).

Das CEA ist hauptsächlich auf den Adenokarzinomen des Gastrointestinaltraktes exprimiert, das N-CAM befindet sich auf Tumoren, die vom Neuroektoderm abgeleitet sind und das PEM kommt hauptsächlich auf Mamma- und Ovarialkarzinomen vor. Diese eben genannten TAA stellen hochmolekulare Glykoproteine dar, die für das murine Immunsystem eine Vielzahl von immunogenen Epitopen tragen. Vergleichende immunhistochemische Untersuchungen auf menschlichen kryopräservierten Geweben belegen, daß die Spezifität eines MAK, der mit seinem Idiotyp (V-Region) ein Epitop I auf einem TAA erkennt, eine andere Gewebebindung zeigen kann, als ein MAK der ein Epitop II erkennt (Buchegger et al. (1984), Int. J. Cancer 33: 643 - 649).

Diese Beobachtung kann vielschichtige Ursachen haben: Kryptizität von Epitopen in bestimmten Geweben, kreuzreaktive Epitope auf unterschiedlichen Antigenen, Konformationsänderungen von Antigenen bei der Sekretion aus Geweben ins Plasma (Bosslet et al (1988), Eur. J. Nucl. Med. 14: 523 - 528) etc. Hieraus kann gefolgert werden, daß die Spezifität eines MAK nicht eindeutig durch die Definition des erkannten Antigens gegeben ist, sondern durch die exakte Beschreibung der V-Region des MAK in Verbindung mit seiner immunhistochemischen Spezifität auf menschlichen kryopräservierten Geweben und seiner Serumspezifität mit zirkulierenden TAA-Strukturen in humanem Serum oder Plasma.
So sind z. B. eine Reihe von MAK gegen das von Shimizu aus humaner Milch (Shimizu, M. und Yamauchi, K. (1982), J. Biochem. 91, 515 - 524) isolierte PEM entwickelt worden, die an verschiedene Epitope binden-und demzufolge unterschiedliche Eigenschaften haben (Girling et al. (1989), 43, 1072 - 1076, Taylor-Papadimitriou et al. (1981), Int. J. Cancer, 28, 17 - 21).

Bestimmte anti-PEM MAK (HMFG 1,2) zeigen eine starke Reaktion mit humanen Mamma- u. Ovarialkarzinomen, reagieren aber auch signifikant mit menschlichen Normalgeweben (Taylor-Papadimitriou et al. (1981)). Andere MAK (SM 3, Girling et al (1989)) reagieren schwächer und heterogener mit Mamma- u. Ovarialkarzinomen, binden dafür aber nicht signifikant an menschliche Normalgewebe. Überraschenderweise ist es uns gelungen, einen anti-PEM-MAK zu erzeugen, der stark mit Mamma-, Ovarial-, Prostata-Karzinomen sowie Adenokarzinomen der Lunge reagiert, nur schwach an menschliche Normalgewebe bindet und zusätzlich in der Lage ist, PEM aus humanem Serum oder Plasma sehr spezifisch nachzuweisen. Verfahren zur immunchemischen Bestimmung von Antigen sind dem Fachmann bekannt (siehe Gosling (1990), Clin. Chem. 36/8, 1408 - 1427). Im wesentlichen werden dabei zwei Klassen unterschieden: homogene Assays, wie z. B. partikelverstärkte Nephelometrie und Turbidimetrie und heterogene, auch als Festphasenassays bezeichnete, Verfahren.
Festphasenassays sind so aufgebaut, daß das Analytantigen durch einen festphasengebundenen Fängerantikörper aus der zu untersuchenden Probe heraus immobilisiert wird und das immobilisierte Antigen durch einen zweiten, mit einem nachweisbaren Markierungsteil versehenen Antikörper (Konjugat) nachgewiesen wird. Solche nachweisbaren Markierungen sind dem Fachmann bekannt und sind z. B. Enzyme, chemilumineszente oder elektrochemilumineszente, radioaktive oder auch farbige Label.

Die Hybridomazellinie BW 835, die den monoklonalen Antikörper BW 835 produziert, wurde am 11.10.91 bei der DSM, Deutsche Sammlung von Mikroorganismen und Zellkulturen GmbH, Mascheroder Weg 1b, D-3300 Braunschweig, unter der Nummer DSM ACC2022 hinterlegt.

Als Antikörper im Sinne dieser Erfindung werden auch Antikörperfragmente verstanden.

Die Herstellung und die Eigenschaften dieses MAK (BW 835) sind im folgenden beschrieben:
Der MAK wurde durch Immunisierung von Balb/c-Mäusen mit den MCF-7 und SW-613 Mammakarzinomzell-Linien nach Literatur-bekannten Methoden (EP-A2-0 141079) erzeugt.

Die Verteilung des durch den MAk BW 835 definierten Epitops auf kryopräservierten humanen Karzinomen bzw. menschlichen Normalgeweben ist in Tabelle I a, b vergleichend zu dem MAK SM-3 (Girling et al. 1989) gezeigt. Die Daten basieren auf einem immunhistochemischen Nachweis mit der APAAP-Technik (Cordell et al. (1984), J. Histochem. Cytochem. 32, 219). Es ist deutlich zu erkennen, daß MAK BW 835 mit allen 15 Mammakarzinomen aus 15 getesteten Mammakarzinomen stark reagiert, wogegen der MAK SM-3 nur 11 aus 15 Mammakarzinomen nachweist.

Beim Ovarialkarzinom reagiert der MAK BW 835 stark mit 6 von 8 getesteten Tumoren, der MAK SM-3 mit einigen wenigen Zellen bei 6 aus 8 Karzinomen. Bei allen weiteren getesteten CA-Typen, vor allem den Adenokarzinomen der Lunge und den Prostatakarzinomen zeigt MAK BW 835 eine quantitativ stärkere Reaktion. Diese Daten zeigen, daß MAK BW 835 mehr Karzinome mit einer quantitativ stärkeren Reaktion als der Literaturbekannte MAK SM-3 nachweist.

Die Bindung des MAK BW 835 an menschliches Normalgewebe ist in Tabelle I b gezeigt. Auch hier wurde die APAAP-Technik zur Erstellung der Daten eingesetzt. Das durch den MAK BW 835 definierte Epitop ist auf dem Gangepithel der Mamma und dem Gangepithel des Pankreas signifikant exprimiert, auf dem Deckepithel der Lunge, einigen Nervenfasern sowie den Sammelkanälchen und den Glomeruli der Niere schwach exprimiert. Alle sonstigen getesteten Normalgewebe sind negativ.

Aufgrund seiner hohen Selektivität kann der MAK BW 835 auch als Induktor für internal Image anti-Paratop MAK verwendet werden. Solche MAK könnten als Epitopvakzine zur Therapie humaner Tumore eingesetzt werden.

Der Nachweis, daß das von dem MAK BW 835 definierte Epitop auf dem durch den MAK SM-3 definierten PEM sitzt, wurde mittels eines Doppeldeterminantenassays überprüft.

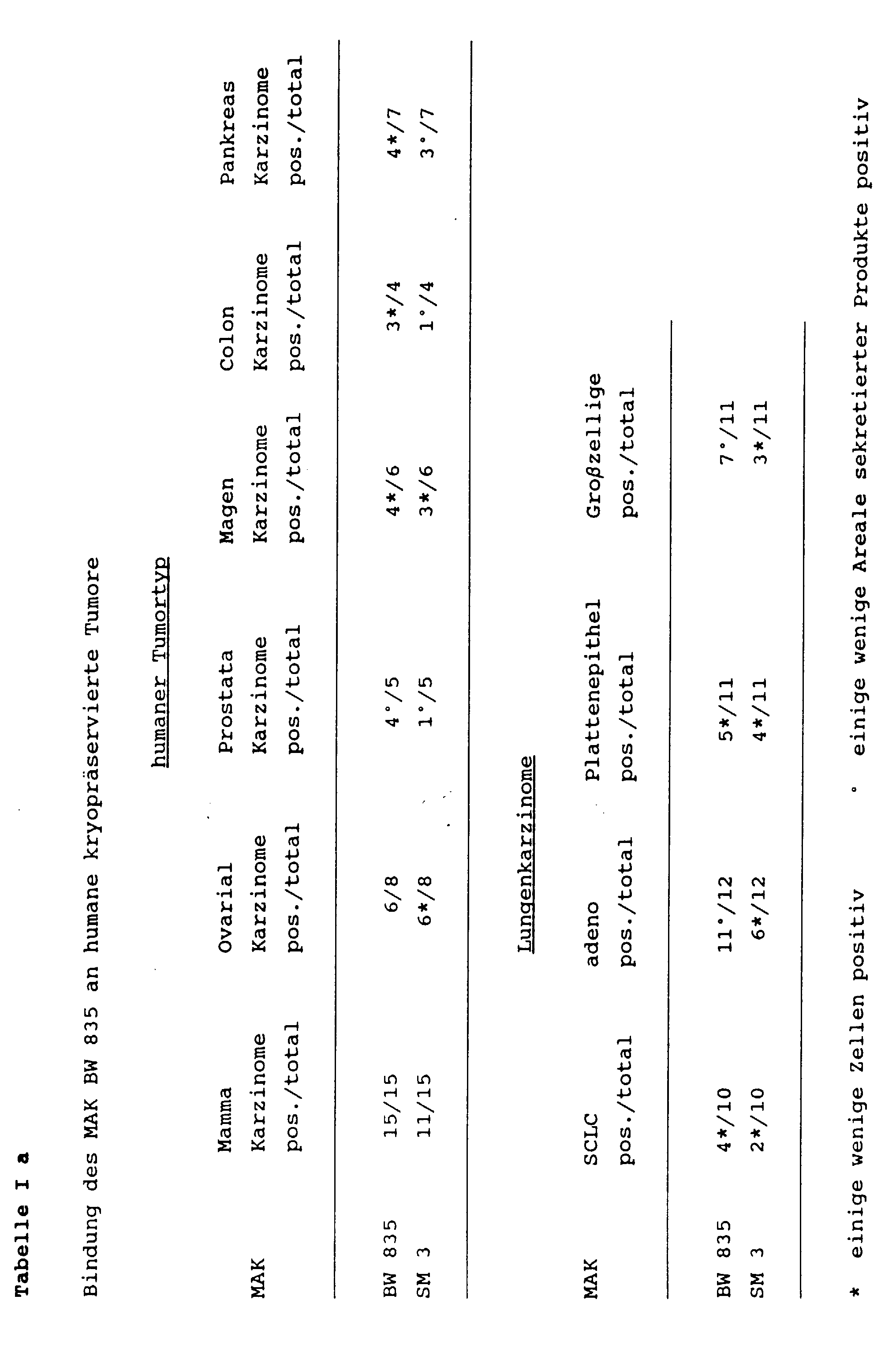

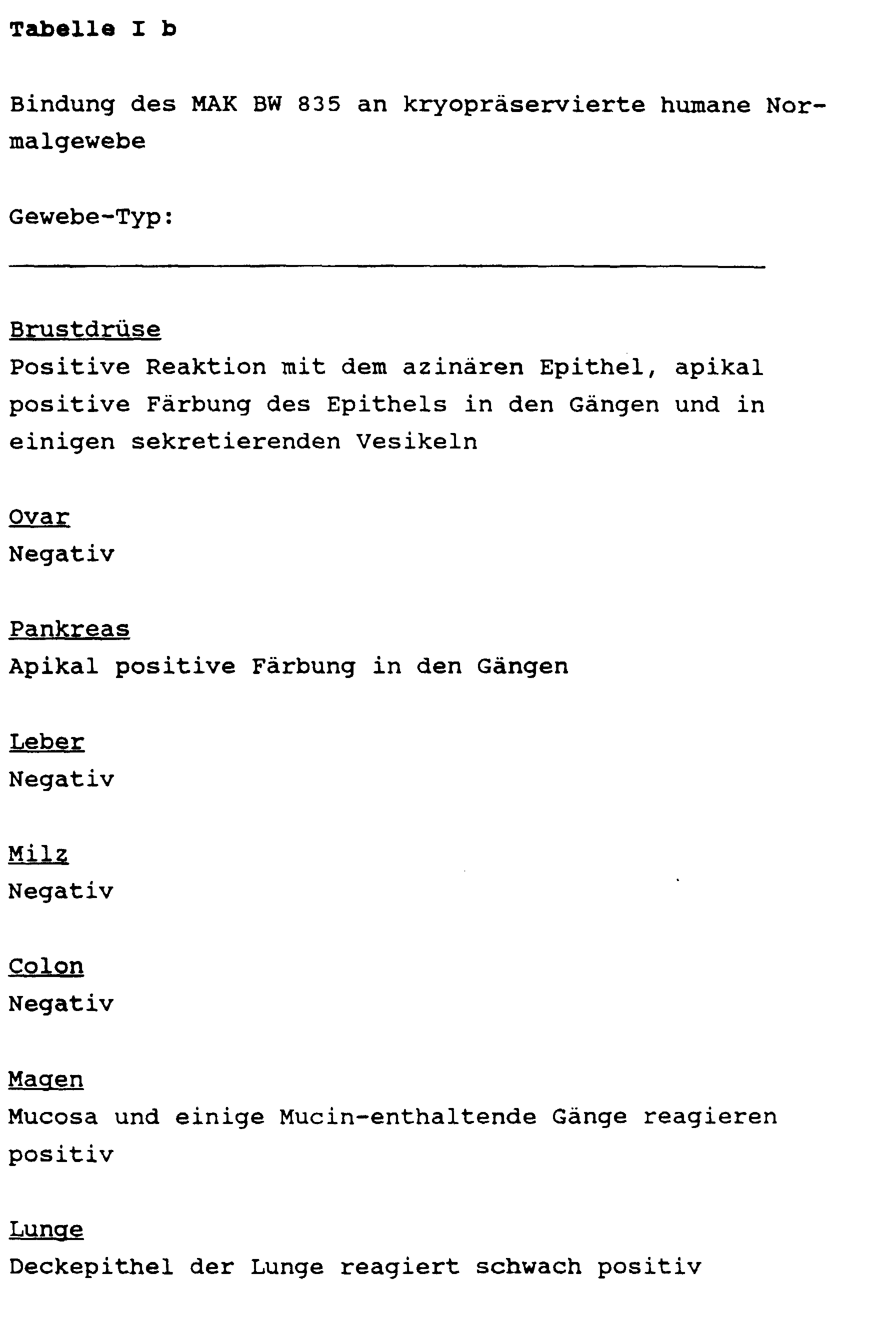

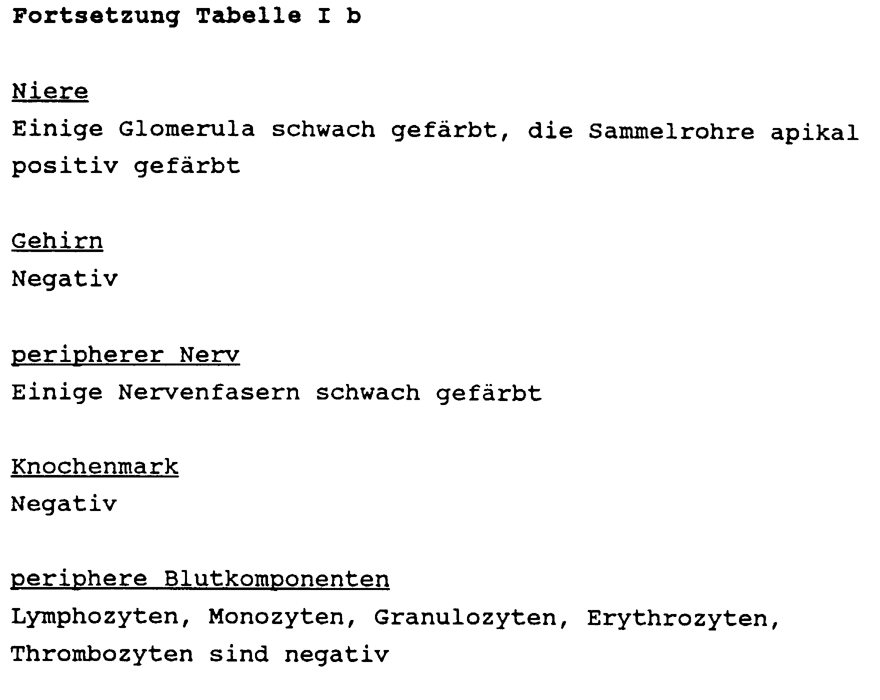

Der als Fänger benutzte MAK BW 835 war in der Lage, aus Zellkulturüberständen der T47 Zell-Linie ein Antigen zu fangen, welches durch den Enzym-markierten MAK SM-3 nachweisbar war. Desweiteren färben beide MAK im Westernblot Moleküle, die der Molekulargewicht-Position des PEMs entsprechen.

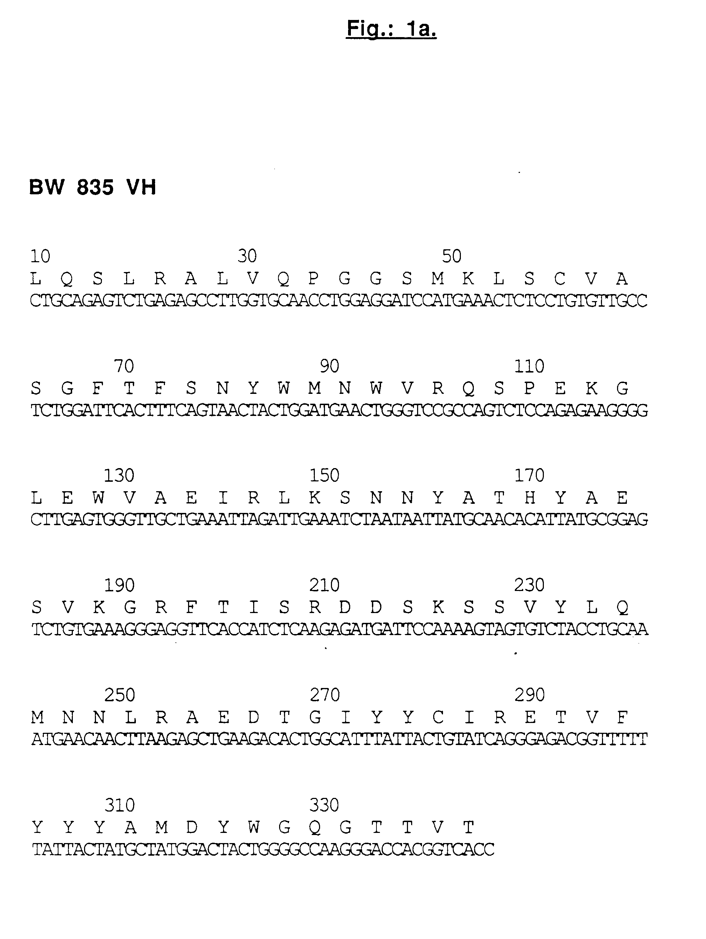

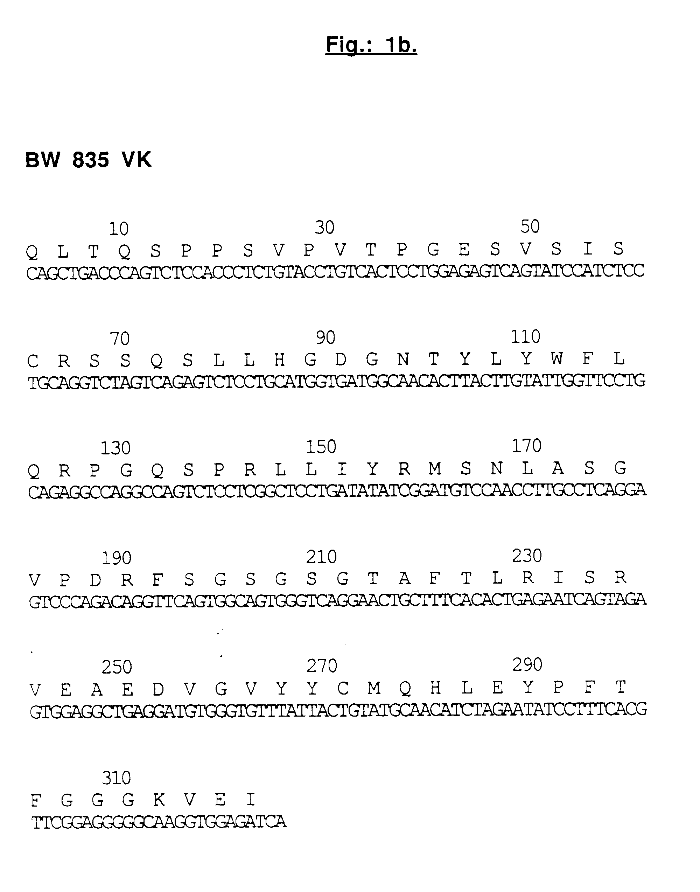

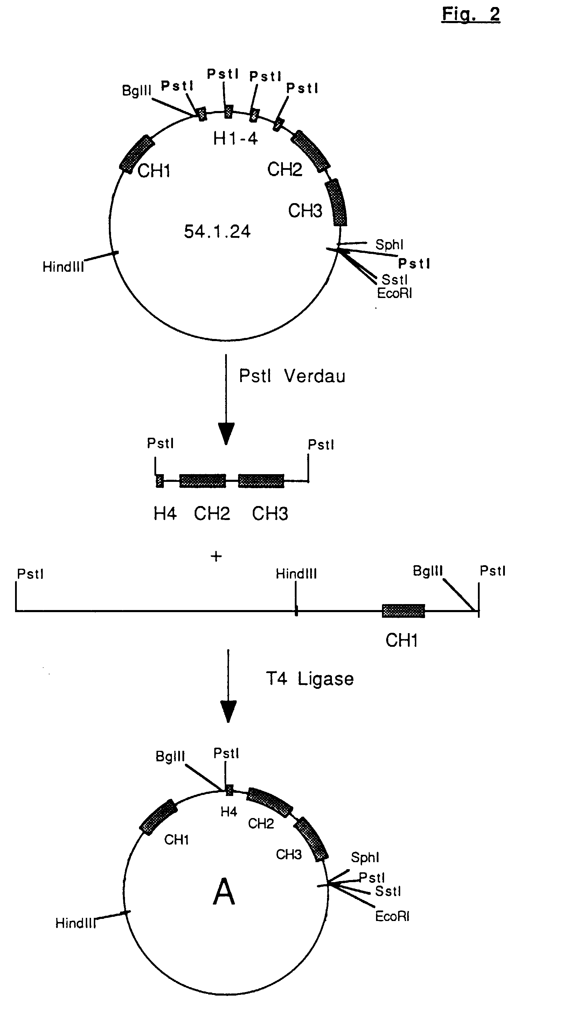

Nachdem die immunologischen Spezifitätsdaten des MAK BW 835 definiert waren, wurde aus 10⁸ Hybridomazellen, welche den MAK BW 835 sekretieren, die mRNA isoliert, nach der von Orlandi et al. (1989), Proc.Natl.Acad.Sci. USA: 86, 3833 - 3837 beschriebenen Methode die V-Gene der schweren und leichten Ketten des MAK BW 835 isoliert und nach der von Sanger et al. (1977), Proc. Natl. Acad. Sci., USA: 74, 5463 - 5467 beschriebenen Methode die Nukleinsäuresequenz der essentiellen Bereiche der V-Gen Exons bestimmt (Fig. 1a, b).

Bei der wiederholten hochdosierten Anwendung von MAK murinen Ursprungs, wie zum Beispiel dem MAk BW 835, für die in vivo-Therapie beim Menschen kann es zu einer Immunisierung der Patienten kommen. Sie können nach etwa 10 - 14 Tagen humane anti-Maus Immunglobulin-Antikörper (HAMA) bilden (Miller et al.,(1983), Blood, 62, 988; Joseph et al.,(1988), European Journal of Nuclear Medicine, 14, 367). Diese HAMAs können die Pharmakokinetik und Pharmakodynamik des MAk ungünstig beeinflussen und die Fortführung der Behandlung erschweren.

Um die Immunogenität von xenogenen Antikörpern so weit wie möglich zu reduzieren, wurde eine Technik entwickelt, bei der nur die CDR-Loops der V_{L}- und V_{H}-Domänen der xenogenen Antikörper auf V_{L}- und V_{H}-Domänen von menschlichen Antikörpern übertragen werden (Jones, P.T., et al.,(1986), Nature, 321, 522) (EP-A-87302620, G. Winter), ein Vorgang der als "Humanisierung" bezeichnet wird und sich auf der Ebene der V_{H}- und V_{L}-Gene abspielt.

Die technische Durchführung der Humanisierung eines Antikörpers gliedert sich im wesentlichen in drei Abschnitte: die Klonierung und Nukleinsäuresequenzanalyse der spezifischen V_{H}- und V_{L}-Gene, den computerunterstützten Entwurf der synthetischen Oligonukleotide für die Übertragung der CDR-Loops auf die menschlichen V_{H}- und V_{L}-Domänen und der übertragung der CDR-Loops auf menschliche V_{H}- und V_{L}-Domänen mittels spezifischer Mutagenese. (Riechmann, L., et al.,(1988), Nature, 332, 323; Verhoeyen, M., et al.,(1988), Science, 239, 1534).

Eine solche Humanisierung kann auch bei dem MAk BW 835 durchgeführt werden, um seine in vivo Anwendbarkeit zu verbessern. Dabei würden die authentischen CDR Regionen der BW 835 V_{H}- und V_{L}-Domänen (definiert nach Kabat, E. A., et al. (1987) Sequences of Proteins of Immunological Interest. fourth edition, US Dept. of Health und Human Services, US Government Printing Office) oder CDR Regionen mit wenigen veränderten Aminosäuren auf menschliche V_{H}- und V_{L}-Domänen übertragen, wobei einzelne Aminosäuren der zwischen den CDR Regionen gelegenen Framework Bereiche von dem Maus Antikörper auf den humanisierten Antikörper übernommen werden können, um die Antigenbindungseigenschaften des MAK BW 835 in der humanisierten Form möglichst unverändert zu erhalten.

Die variablen Domänen des humMAK BW 835 bestehen demnach aus den authentischen oder an einigen Stellen veränderten Framework-Regionen der variablen Domänen eines menschlichen MAk, auf welche die authentischen oder an wenigen Aminosäurepositionen veränderte CDR-Regionen des Maus MAK BW 835 transplantiert wurden.

In den folgenden Beispielen werden die Arbeitsschritte beschrieben, welche für die Klonierung und Nukleinsäuresequenzanalyse der V-Gene und für die Expression der BW 835 Spezifität als chimärer MAK erforderlich waren. Die in den Beispielen 1 - 12 verwendeten Techniken wurden, wenn nicht anders angezeigt, aus Molecular Cloning, a Laboratory Manual; Sambrook, Fritsch, Maniatis; Cold Spring Harbor Laboratory, 1982 (S. 11 - 44, 51 - 127, 133 - 134, 141, 146, 150 - 167, 170, 188 - 193, 197 - 199, 248 - 255, 270 - 294, 310 - 328, 364 - 401, 437 - 506) und aus Molecular Cloning, A Laboratory Manual, Second Edition; Sambrook, Fritsch, Maniatis; Cold Spring Harbor Laboratory Press, 1989 (S. 16.2 - 16.22, 16.30 - 16.40, 16.54 - 16.55) entnommen.

### Beispiel 1:

Der Plasmidklon 54.1.24, der das humane IgG₃ C-Gen trägt (Fig. 2) (DE-A1-38 25 615, Fig. 2) wurde mit PstI gespalten. Der dabei entstehende Vektor wurde mit dem größten der entstehenden PstI Insert Fragmente ligiert und in Bakterien transformiert. Durch Restriktionsanalyse und Nukleinsäuresequenzbestimmung wurde der Plasmidklon A identifiziert, der ein humanes IgG₃ C-Gen trägt, bei welchem die H1, H2 und H3 Exons deletiert sind (IgG3 ).

### Beispiel 2:

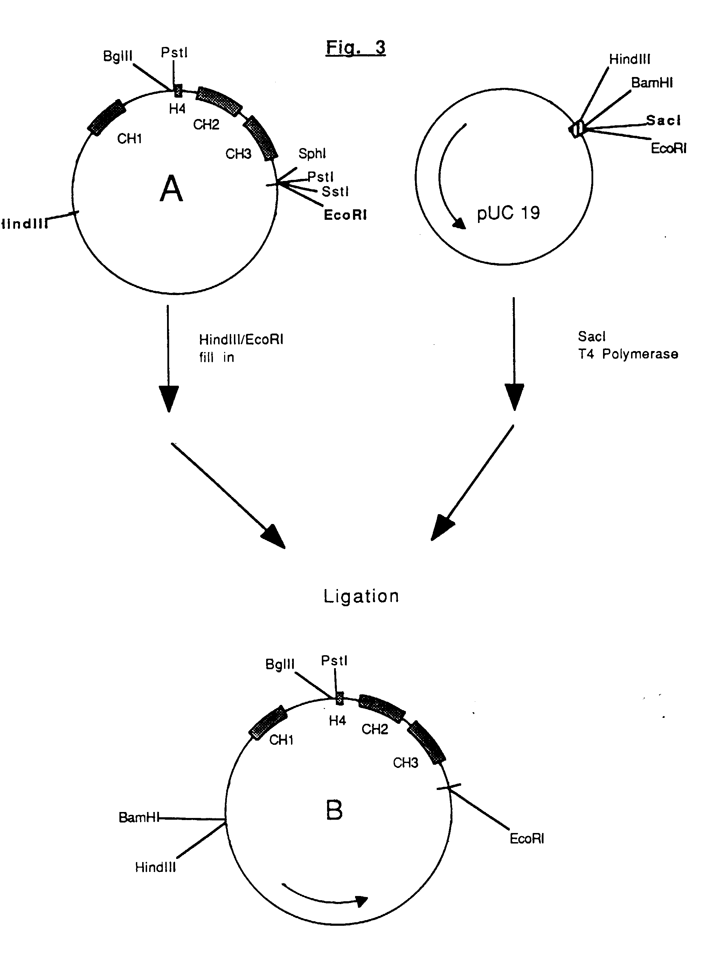

Der Plasmidklon A wurde mit HindIII und EcoRI gespalten, die Enden mit Klenow Polymerase aufgefüllt, das IgG₃ Insert isoliert und in einen mit SstI gespaltenen und mit Hilfe von T₄ Polymerase mit glatten Enden versehenen pUC19 Vektor ligiert. Durch Restriktionskartierung und Nukleinsäuresequenzanalyse wurde ein Plasmidklon B identifiziert, bei dem das IgG₃ Gen so orientiert ist, daß sich am 5' Ende die HindIII und am 3' Ende die EcoRI Schnittstelle des pUC19 Polylinkers befinden (Fig. 3).

### Beispiel 3:

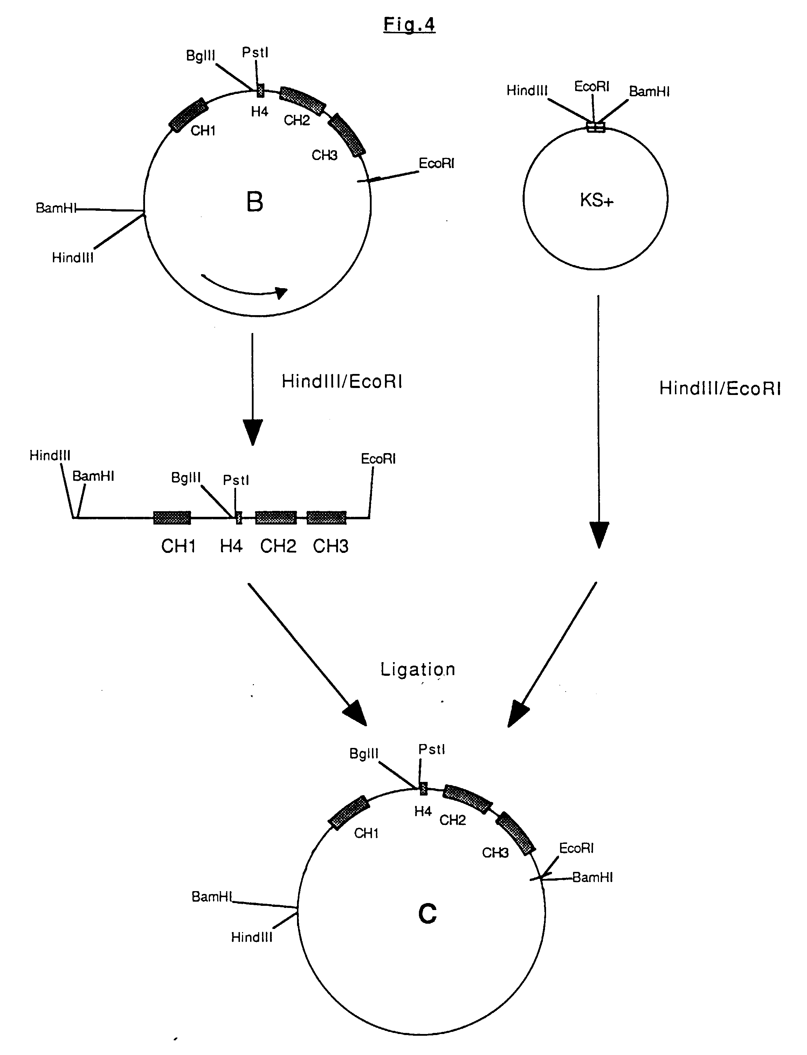

Das Plasmidklon B wurde mit EcoRI und HindIII gespalten, das IgG₃ Insert isoliert und in einen ebenfalls HindIII und EcoRI gespaltenen KS+ Plasmidvektor (pBluescriptII KS+; Stratagene, La Jolla, CA) ligiert. Es wurde der Phasmidklon C isoliert, bei dem das IgG₃ Gen am 5' und am 3' Ende von einer BamHI Schnittstelle flankiert wird (Fig. 4).

### Beispiel 4:

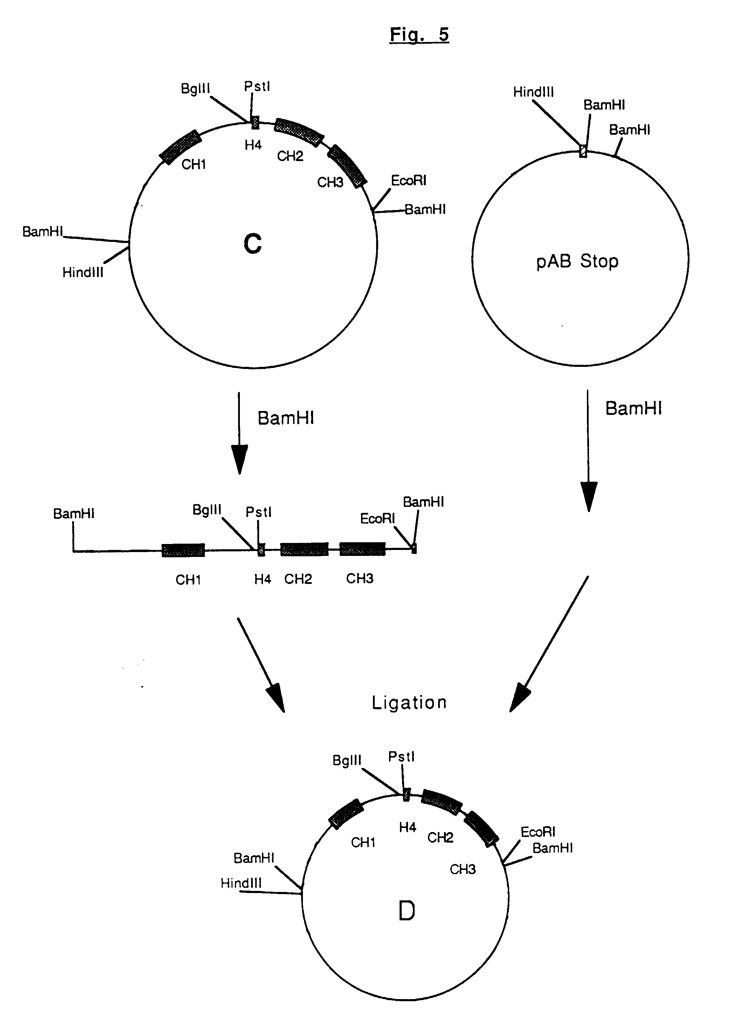

Der Phasmidklon C wurde mit BamHI gespalten, das IgG₃ Insert isoliert und in den ebenfalls mit BamHI gespaltenen Expressionsvektor pABStop (Wirth et al. (1988), Gene, 73, 419 - 426) ligiert. Es wurde das Expressionsplasmid D identifiziert, welches das IgG₃ C-Gen in der in Fig. 5 gezeigten Orientierung enthält. Bei dieser Klonierung verliert der pABStop Vektor das zwischen den beiden BamHI Schnittstellen gelegene SV40 Stop und Polyadenylierungssignal.

### Beispiel 5:

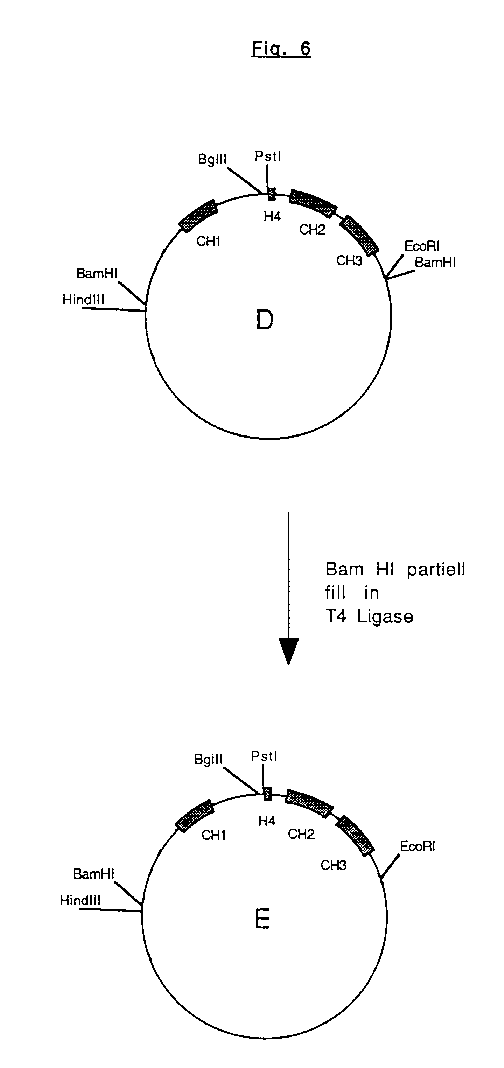

Das Expressionsplasmid D wurde mit BamHI partiell gespalten, die Enden mit Klenow Polymerase aufgefüllt und religiert. Es wurde das Expressionsplasmid E isoliert, bei dem die BamHI Schnittstelle 3' vom IgG₃ Gen zerstört ist (Fig. 6).

### Beispiel 6:

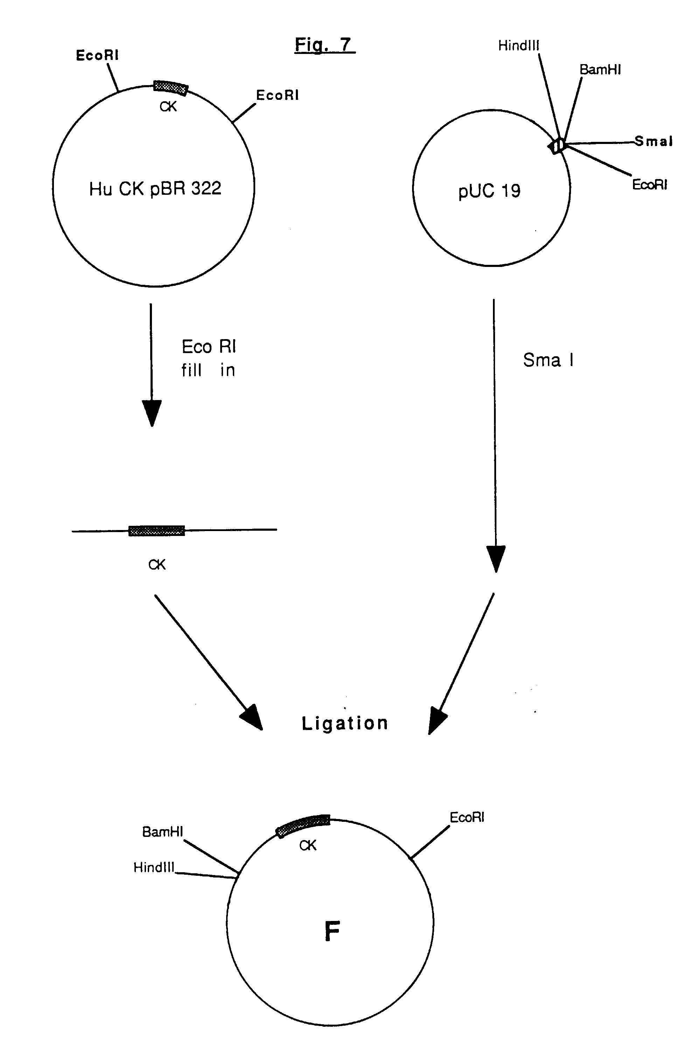

Das humane C-Kappa Gen (Hieter et al. (1982), J. Biol. Chem., 257, No. 3, 1516 - 1522) wurde als EcoRI Fragment in pBR 322 kloniert von Prof. P. Leder, Harvard Medical School, erhalten. Der pBR322 Vektor wurde mit EcoRI gespalten, die EcoRI Schnittstellen aufgefüllt, das C-Kappa Insert isoliert und in einen mit SmaI gespaltenen pUC19 Vektor ligiert. Es wurde der Plasmidklon F isoliert, bei dem das C-Kappa Gen am 5' Ende von einer HindIII nach einer BamHI Schnittstelle und am 3' Ende von einer EcoRI Schnittstelle flankiert wird (Fig. 7).

### Beispiel 7:

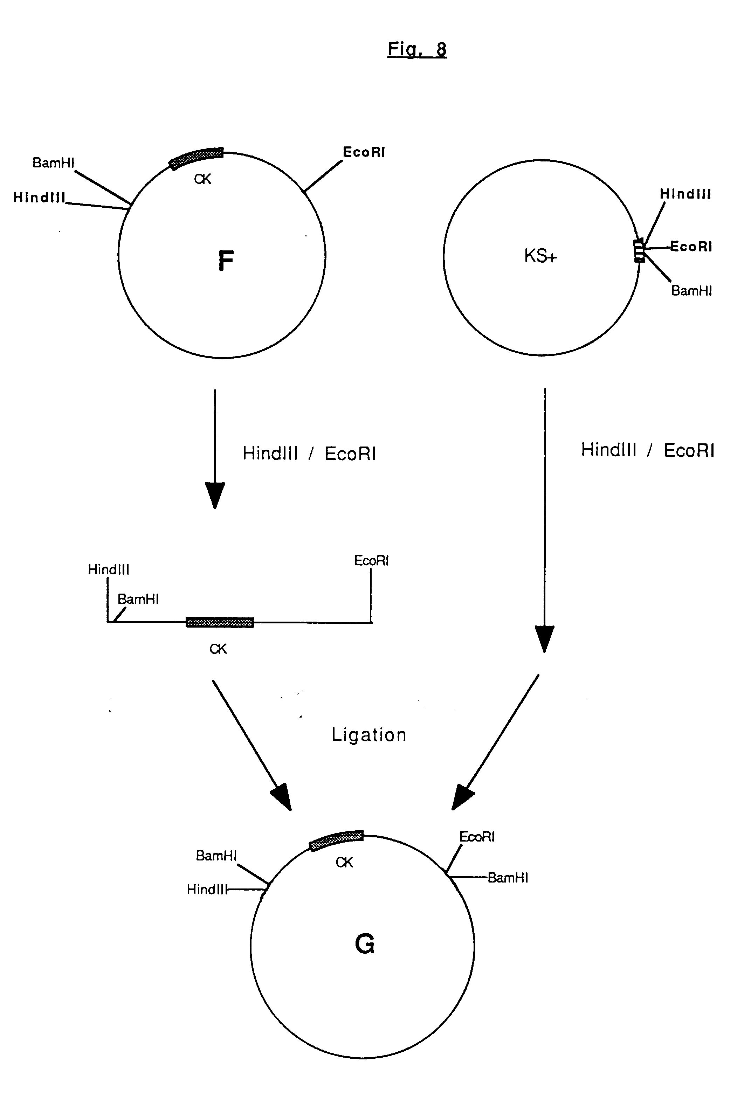

Der Plasmidklon F wurde mit HindIII und EcoRI gespalten, das C-Kappa Insert isoliert und in einen HindIII/EcoRI gespaltenen KS+ Phasmid kloniert. Es wurde der Phasmidklon G isoliert, bei dem das C-Kappa Insert am 5' und am 3' Ende von einer BamHI Schnittstelle flankiert wird (Fig. 8).

### Beispiel 8:

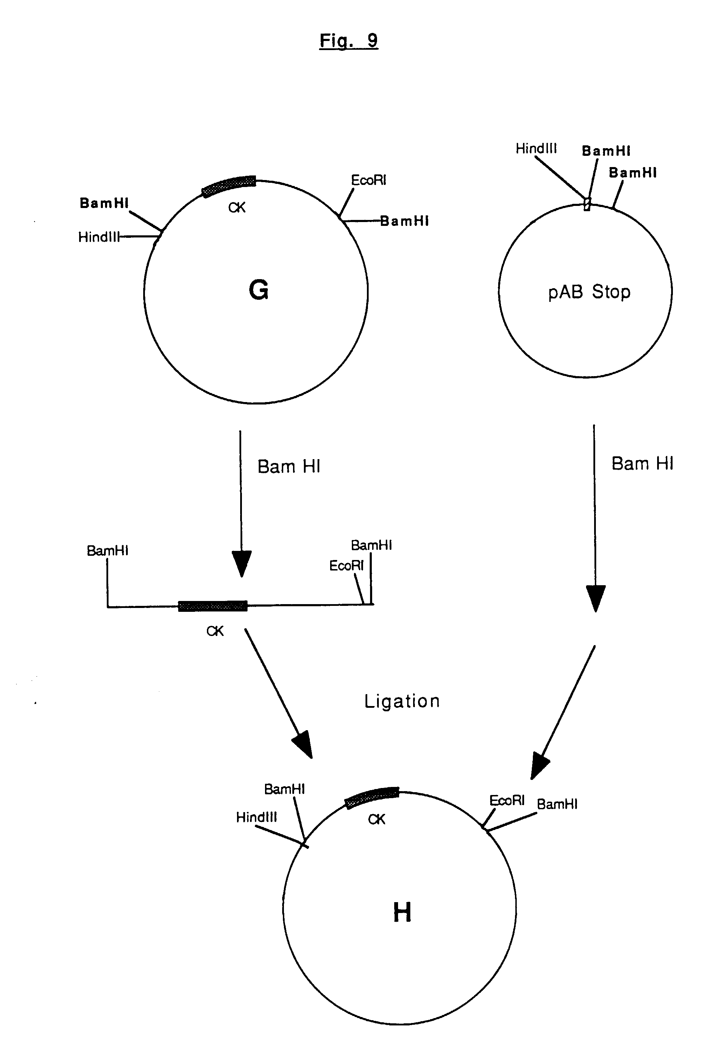

Der Phasmidklon G wurde mit BamHI gespalten, das C-Kappa Insert isoliert und in einen mit BamHI gespaltenen pAB Stop Vektor kloniert. Durch Restriktionskartierung und Nukleinsäuresequenzanalyse wurde der Klon H identifiziert, bei dem das C-Kappa Gen so orientiert ist, daß an seinem 5' Ende die HindIII Schnittstelle des pAB Stop Vektors liegt (Fig. 9).

### Beispiel 9:

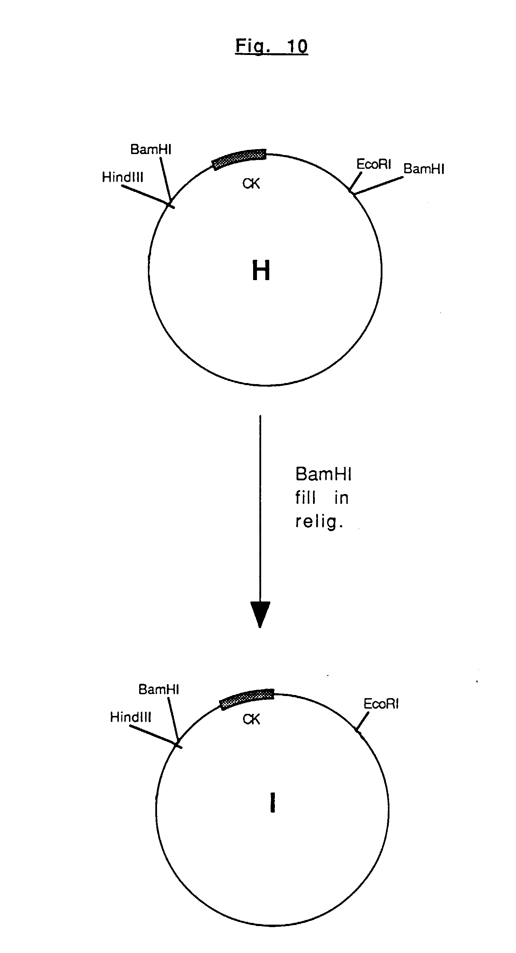

Der Klon H wurde mit BamHI partiell gespalten, die Restriktionsenden aufgefüllt und religiert. Durch Restriktionskartierung wurde der Klon I identifiziert, bei dem die BamHI Schnittstelle 3' des C-Kappa Gens zerstört ist (Fig. 10).

### Beispiel 10:

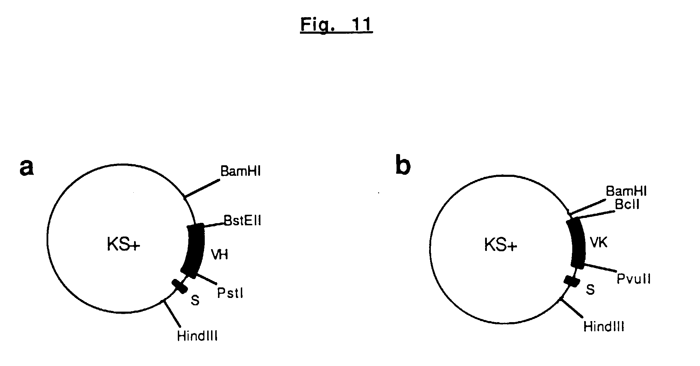

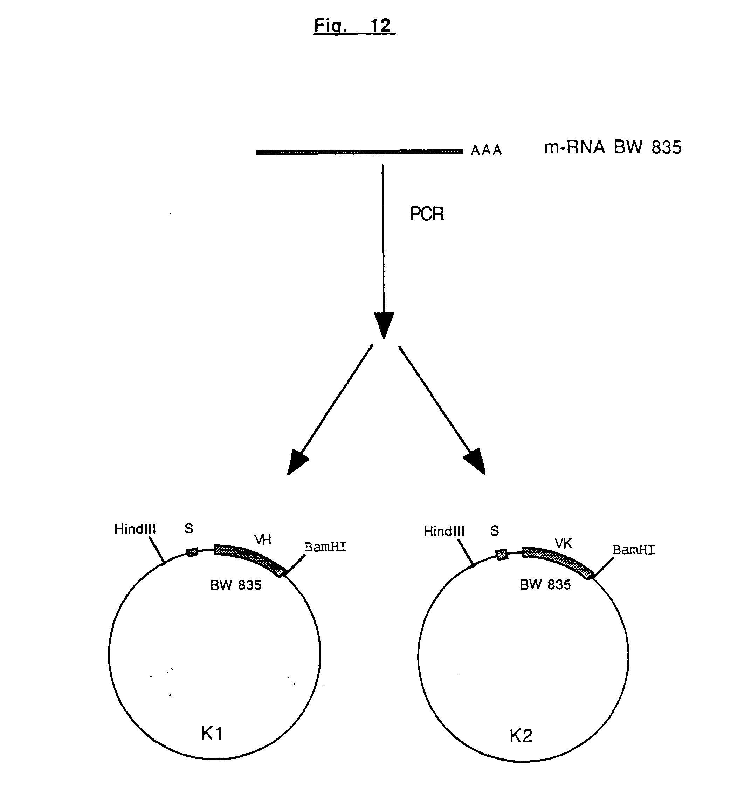

Die V_{H} und V_{K} Gene des MAk BW 835 wurden nach Orlandi et al. (1989) mittels PCR Technik und spezifischer Oligonukleotide amplifiziert und in KS+ Vektoren kloniert (Güssow and Seemann (1991), Methods in Enzymology, Vol. 203) welche V_{H} und V_{K} Gene mit geeigneten Restriktionsschnittstellen enthielten (Fig. 11 a für V_{H} und b für V_{K}). Es wurden die Klone K1 und K2 isoliert, welche die V_{H} (K1) und V_{K} (K2) Gene des MAk BW 835 enthalten (Fig. 12).

### Beispiel 11:

Aus den Klonen K1 und K2 wurde die Nukleinsäuresequenz der V_{H} und V_{K} Gene des MAK BW 835 nach der Methode von Sanger et al. (1977) bestimmt (Fig. 1a, b). Aus dieser Sequenz können nach der von Saragovi et al. (Saragovi et al. (1991), Science 253, 792-795) beschriebenen Methode Mimetika die auf den CDRs basieren, erzeugt werden.

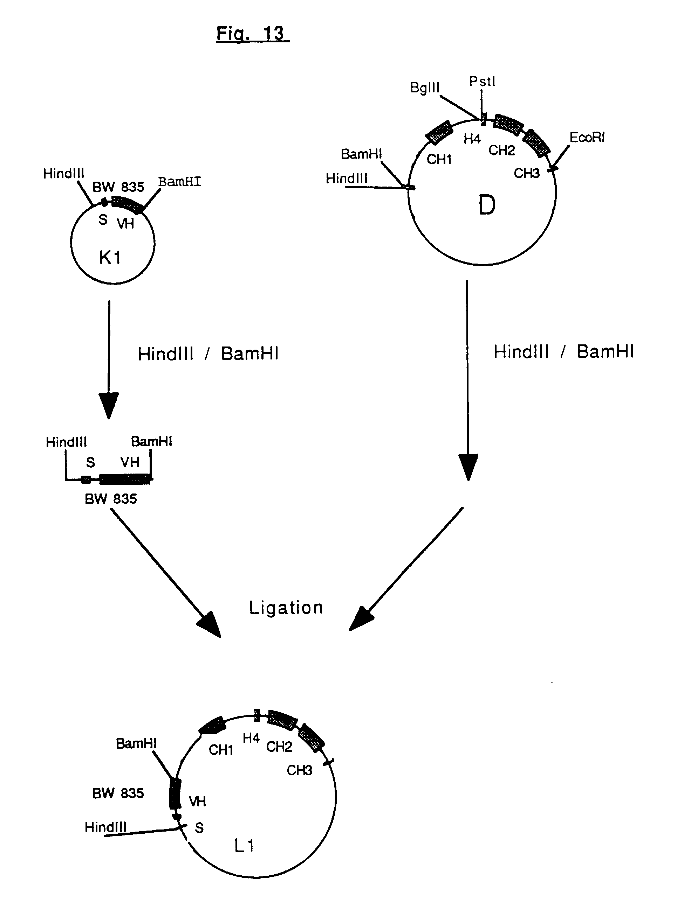

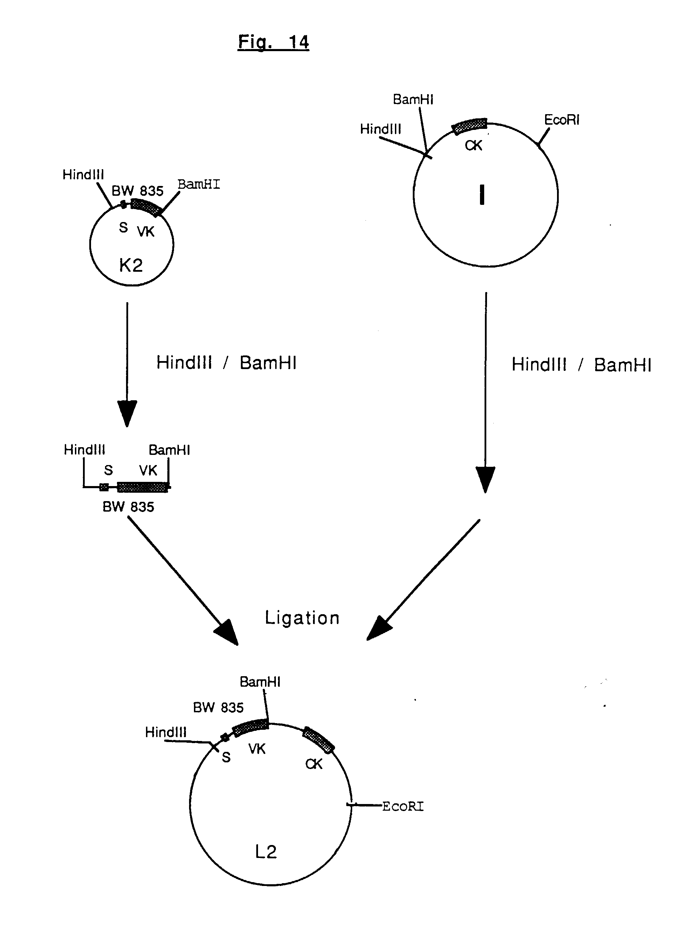

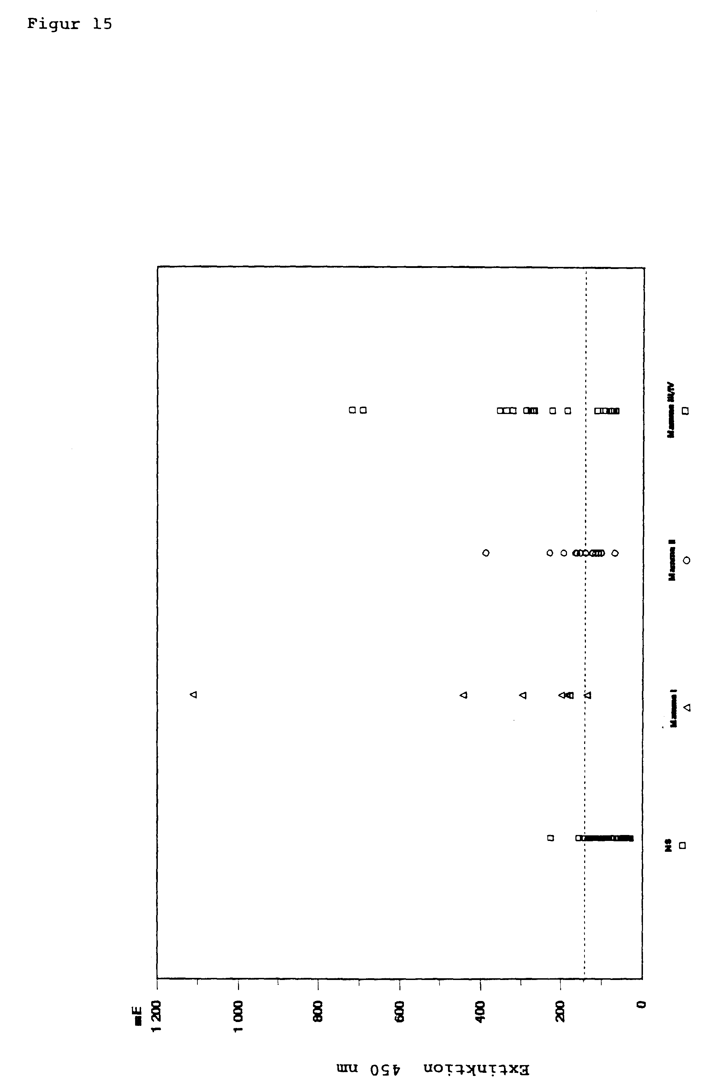

Desweiteren wurden aus den Klonen K1 und K2 mit Hilfe der Restriktionsendonukleasen HindIII und BamHI die V_{H} und V_{K}-Gen Inserts ausgeschnitten und in die ebenfalls mit HindIII und BamHI gespaltenen Vektoren D (V_{H}) und I (V_{K}) kloniert. Es wurden die Expressionsvektoren L1 und L2 isoliert, welche Immunglobulin schwere (L1) (Fig. 13) und leichte (L2) (Fig. 14) Ketten Gene mit den V Genen des MAK BW 835 enthalten. Die Expressionsvektoren L1 und L2 können für die Expression eines chimären MAK mit der Spezifität des MAK BW 835 verwendet werden.

Die Beispiele 12 und 13 sollen die Verwendung des MAK BW 835 zur Serum-Diagnostik von malignen Tumoren erläutern.

### Beispiel 12:

Der MAK BW 835 wurde nach dem Fachmann bekannten Verfahren (Tijssen, P., "Practice and theory of enzyme immunoassay" Elsevier (1988), 297 - 328) adsorptiv an die Wandung von Vertiefungen von Mikrotitrationsplatten (NUNC) gebunden. In die so präparierten Vertiefungen wurden zu je 100 µl Puffer (OSND, Behringwerke) je 10 µl Probe hinzupipettiert und 2 Stunden bei +37°C inkubiert. Nach dreimaligem Waschen mit verdünntem Enzygnost Waschpuffer (OSEW, Behringwerke) wurden in die einzelnen Vertiefungen je 100 µl MAK BW 835 (1 µg/ml), der nach den bekannten Verfahren mit Peroxidase konjugiert wurde, eingefüllt. Der folgende 2stündige Inkubationsschritt bei +37°C wurde mit einem dreimaligen Waschzyklus abgeschlossen. Für den dritten Inkubationsschritt bei Raumtemperatur wurden anschließend je 100 µl einer Puffer/Substrat-Chromogenlösung (OUVG/OUVF, Behringwerke) in die Vertiefungen pipettiert und die Enzymreaktion nach 30 Minuten mit 100 µl Enzygnost-Stopplösung (OSFA, Behringwerke) beendet. Die Extinktion der Proben wurde bei 450 nm ermittelt.

### Ergebnis:

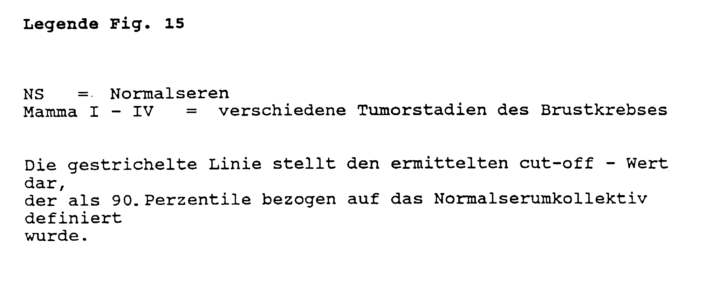

Die so ermittelten Extinktionswerte entsprechen in ihrer Höhe der Konzentration des Antigens in der Probe. Die Konzentration des durch die spezifische Bindung des MAK BW 835 definierten Antigens im Serum oder Plasma von Patienten mit malignen Tumoren ist im Vergleich zu der bei gesunden Patienten deutlich erhöht (Fig. 15). Dies gilt besonders für Mamma-Karzinompatienten im Spätstadium, aber auch überraschenderweise für solche in Mammafrühstadien, die von anderen kommerziellen Tumormarkertests zur Detektion von Brustkrebs-assoziierten Antigenen im Serum (z. B. CA 15-3) vergleichsweise signifikant häufiger falsch negativ gefunden werden und somit über alles für die beschriebene homologe Version bessere Sensitivitäten ermittelt werden konnten.

### Beispiel 13:

Zur Detektion von PEM im Serum können im Doppeldeterminantenassay in Kombination mit dem MAK BW 835 auch andere peroxidasemarkierte Antikörper verwendet werden, die weitere Epitope auf dem vom MAK BW 835 definierten, tumorassoziierten Antigenen erkennen. Hierzu wurde z.B. ein nach Beispiel 12 analoger Test durchgeführt, der den literaturbekannten DF3-Antikörper (Kufe et al (1984), Hybridoma 3, 223) in peroxidase markierter Form (CA 15-3-Test, Boehringer Mannheim) als Konjugatkomponente verwendet.

### Ergebnis:

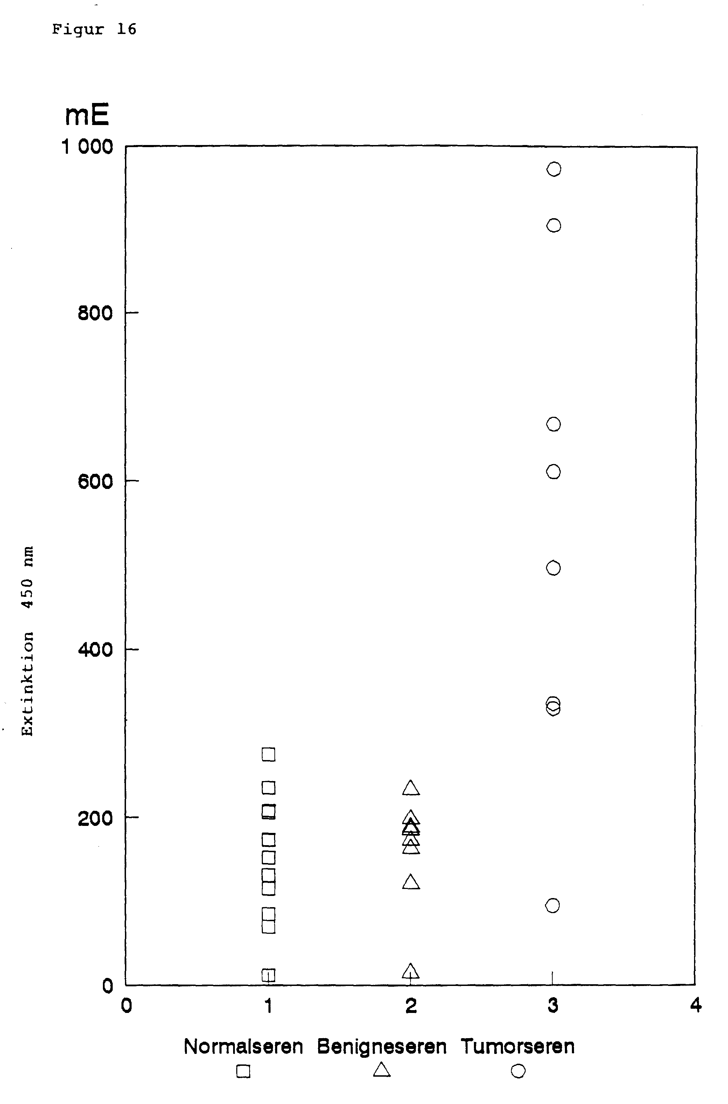

Die Verwendung z. B. des DF3-POD als Konjugatkomponente in Ergänzung zu dem festphasengebundenen BW 835 erbrachte deutlich erhöhte Serumwerte für die Tumorseren im Vergleich zu einem Normalserumkollektiv bzw. Patienten mit gutartigen Erkrankungen, was nochmals das Potential des MAK BW 835 als spezifische Komponente für einen Tumormarkertest unterstreicht (Fig. 16).

## Patentansprüche (Patentansprüche für folgende(n) Vertragsstaat(en): AT, BE, CH, LI, DE, DK, FR, GB, IT, LU, NL, PT, SE)

1. Monoklonale Antikörper gegen ein tumorassoziiertes Antigen, dadurch gekennzeichnet, daß das Antigen von Tumoren vornehmlich aus der Gruppe der Mamma-, Ovarial-, Prostata-Karzinome sowie Adenokarzinome der Lunge stammt, die Antikörper zusätzlich mit "polymorphic epithelial mucin" (PEM) reagieren und daß die Antikörper ein Epitop binden, das durch den monoklonalen Antikörper, der von der Hybridomazellinie DSM ACC2022 produziert wird, gebunden wird.

2. Monoklonaler Antikörper gemäß Anspruch 1, dadurch gekennzeichnet, daß er von der Hybridomazellinie DSM ACC2022 produziert wird.

3. Spezifischer Bindungspartner, dadurch gekennzeichnet, daß er an dasselbe Epitop bindet, wie der monoklonale Antikörper gemäß Anspruch 2.

4. Fusionsproteine mit den antigenbindenden Eigenschaften eines monoklonalen Antikörpers gemäß Anspruch 1.

5. Antikörper gemäß Anspruch 1, dadurch gekennzeichnet, daß die nichtantigenbindenden Teile in der Aminosäuresequenz einer humanen Sequenz entsprechen.

6. Hybridomazellinie, dadurch gekennzeichnet, daß sie monoklonale Antikörper gemäß Anspruch 1 produziert.

7. Hybridomazellinie gemäß Anspruch 6 mit der Hinterlegungsnummer DSM ACC2022.

8. Nukleinsäure mit der Nukleotidsequenz gemäß Fig. 1a oder 1b.

9. Polypeptid mit der Aminosäuresequenz gemäß Fig. 1a oder 1b.

10. Diagnostikum, enthaltend einen monoklonalen Antikörper gemäß Anspruch 1.

11. Antikörper gemäß Anspruch 1 zur Verwendung in einem in-vitro Diagnostikum.

12. Verwendung gemäß Anspruch 11 zur Detektion von tumorassoziierten Antigenen in biologischen Proben.

13. Antikörper gemäß Anspruch 1 zur Verwendung in einem in-vivo Diagnostikum.

14. Antikörper gemäß Anspruch 1 zur Verwendung als Therapeutikum.

## Patentansprüche (Patentansprüche für folgende(n) Vertragsstaat(en): ES)

1. Verwendung von monoklonalen Antikörpern gegen ein tumorassoziiertes Antigen, wobei die monoklonalen Antikörper dadurch gekennzeichnet sind, daß das Antigen von Tumoren vornehmlich aus der Gruppe der Mamma-, Ovarial-, Prostata-Karzinome sowie Adenokarzinome der Lunge stammt, die Antikörper zusätzlich mit "polymorphic epithelial mucin" (PEM) reagieren und daß die Antikörper ein Epitop binden, das durch den monoklonalen Antikörper, der von der Hybridomazellinie DSM ACC2022 produziert wird, gebunden wird.

2. Verwendung gemäß Anspruch 1, dadurch gekennzeichnet, daß der monoklonale Antikörper von der Hybridomazellinie DSM ACC2022 produziert wird.

3. Verwendung eines spezifischen Bindungspartners, der dadurch gekennzeichnet ist, daß er an dasselbe Epitop bindet, wie der monoklonale Antikörper, der von der Hybridomazellinie DSM ACC2022 produziert wird.

4. Verwendung von Fusionsproteinen mit den antigenbindenden Eigenschaften eines monoklonalen Antikörpers nach Anspruch 1.

5. Verwendung gemäß Anspruch 1, dadurch gekennzeichnet, daß die nichtantigenbindenden Teile in der Aminosäuresequenz der Antikörper einer humanen Sequenz entsprechen.

6. Verwendung einer Hybridomazellinie, die monoklonale Antikörper nach Anspruch 1 produziert.

7. Verwendung der Hybridomazellinie mit der Hinterlegungsnummer DSM ACC2022.

8. Verwendung einer Nukleinsäure mit der Nukleotidsequenz gemäß Fig. 1a oder 1b.

9. Verwendung eines Polypeptids mit der Aminosäuresequenz gemäß Fig. 1a oder 1b.

10. Verwendung eines Antikörpers nach Anspruch 1 in einem in-vitro Diagnostikum.

11. Verwendung gemäß Anspruch 10 zur Detektion von tumorassoziierten Antigenen in biologischen Proben.

12. Verwendung eines Antikörpers nach Anspruch 1 zur Herstellung eines in-vivo Diagnostikums.

13. Verwendung eines Antikörpers nach Anspruch 1 zur Herstellung eines Therapeutikums.

## Claims (Claims for the following Contracting State(s): AT, BE, CH, LI, DE, DK, FR, GB, IT, LU, NL, PT, SE)

1. Monoclonal antibodies against a tumor-associated antigen, wherein the antigen derives from tumors principally from the group of carcinomas of the breast, ovaries and prostate, as well as adenocarcinomas of the lung, the antibodies additionally react with polymorphic epithelial mucin (PEM) and wherein the antibodies bind an epitope which is bound by the monoclonal antibody which is produced by the hybridoma cell line DSM ACC2022.

2. A monoclonal antibody as claimed in claim 1, which is produced by the hybridoma cell line DSM ACC2022.

3. A specific binding partner which binds to the same epitope as the monoclonal antibody as claimed in claim 2.

4. Fusion proteins having the antigen-binding properties of a monoclonal antibody as claimed in claim 1.

5. An antibody as claimed in claim 1, wherein the non-antigen-binding parts in the amino-acid sequence correspond to a human sequence.

6. A hybridoma cell line which produces a monoclonal antibody as claimed in claim 1.

7. A hybridoma cell line as claimed in claim 6 having the deposition number DSM ACC2022.

8. A nucleic acid having the nucleotide sequence according to Fig. 1a or 1b.

9. A polypeptide having the amino acid sequence according to Fig. 1a or 1b.

10. A diagnostic aid containing a monoclonal antibody as claimed in claim 1.

11. An antibody as claimed in claim 1 for use in an in vitro diagnostic aid.

12. The use as claimed in claim 11 for detecting tumor-associated antigens in biological samples.

13. An antibody as claimed in claim 1 for use in an in vivo diagnostic aid.

14. An antibody as claimed in claim 1 for use as a therapeutic agent.

## Claims (Claims for the following Contracting State(s): ES)

1. The use of monoclonal antibodies against a tumor-associated antigen, in which the monoclonal antibodies are characterized that the antigen derives from tumors principally from the group of carcinomas of the breast, ovaries and prostate, as well as adenocarcinomas of the lung, the antibodies additionally react with polymorphic epithelial mucin ( PEM ) and the antibodies bind an epitope which is bound by the monoclonal antibody which is produced by the hybridoma cell line DSM ACC2022.

2. The use as claimed in claim 1, wherein the monoclonal antibody is produced by the hybridoma cell line DSM ACC2022.

3. The use of a specific binding partner which binds to the same epitope as the monoclonal antibody which is produced by the hybridoma cell line DSM ACC 2022.

4. The use of fusion proteins having the antigen-binding properties of a monoclonal antibody according to claim 1.

5. The use as claimed in claim 1, wherein the non-antigen-binding parts in the amino-acid sequence of the antibodies correspond to a human sequence.

6. The use of a hybridoma cell line which produces monoclonal antibodies according to claim 1.

7. The use of a hybridoma cell line having the deposition number DSM ACC2022.

8. The use of a nucleic acid having the nucleotide sequence according to Fig. 1a or 1b.

9. The use of a polypeptide having the amino acid sequence according to Fig. 1a or 1b.

10. The use of an antibody according to claim 1 in an in vitro diagnostic aid.

11. The use as claimed in claim 10 for detecting tumor-associated antigens in biological samples.

12. The use of an antibody according to claim 1 for producing an in vivo diagnostic aid.

13. The use of an antibody according to claim 1 for producing a therapeutic agent.

## Revendications (Revendications pour l'(les) Etat(s) contractant(s) suivant(s): AT, BE, CH, LI, DE, DK, FR, GB, IT, LU, NL, PT, SE)

1. Anticorps monoclonaux dirigés contre un antigène associé à des tumeurs, caractérisés en ce que l'antigène est issu de tumeurs choisies principalement dans le groupe des carcinomes mammaire, de l'ovaire, de la prostate ainsi que des adénocarcinomes du poumon, les anticorps en outre réagissent avec la "mucine épithéliale polymorphe" (PEM), et en ce que les anticorps se fixent à un épitope qui est lié par l'anticorps monoclonal qui est produit par la lignée d'hybridomes DSM ACC2022.

2. Anticorps monoclonal selon la revendication 1, caractérisé en ce qu'il est produit par la lignée d'hybridomes DSM ACC2022.

3. Partenaire de liaison spécifique, caractérisé en ce qu'il se fixe au même épitope que l'anticorps monoclonal selon la revendication 2.

4. Protéines de fusion ayant les propriétés de liaison à l'antigène d'un anticorps monoclonal selon la revendication 1.

5. Anticorps selon la revendication 1, caractérisé en ce que les segments ne se liant pas à l'antigène dans la séquence d'aminoacides correspondent à une séquence humaine.

6. Lignée d'hybridomes, caractérisée en ce qu'elle produit des anticorps monoclonaux selon la revendication 1.

7. Lignée d'hybridomes selon la revendication 6, portant le numéro de dépôt DSM ACC2022.

8. Acide nucléique ayant la séquence nucléotidique selon la figure 1a ou 1b.

9. Polypeptide ayant la séquence d'aminoacides selon la figure la ou 1b.

10. Agent de diagnostic, contenant un anticorps monoclonal selon la revendication 1.

11. Anticorps selon la revendication 1, pour utilisation dans un agent de diagnostic in vitro.

12. Utilisation selon la revendication 11, pour la détection d'antigènes associés à des tumeurs dans des échantillons biologiques.

13. Anticorps selon la revendication 1, pour utilisation dans un agent de diagnostic in vivo.

14. Anticorps selon la revendication 1, pour utilisation comme agent thérapeutique.

## Revendications (Revendications pour l'(les) Etat(s) contractant(s) suivant(s): ES)

1. Utilisation d'anticorps monoclonaux dirigés contre un antigène associé à des tumeurs, les anticorps monoclonaux étant caractérisés en ce que l'antigène est issu de tumeurs choisies principalement dans le groupe des carcinomes mammaire, de l'ovaire, de la prostate ainsi que des adénocarcinomes du poumon, les anticorps en outre réagissent avec la "mucine épithéliale polymorphe" (PEM), et en ce que les anticorps se fixent à un épitope qui est lié par l'anticorps monoclonal qui est produit par la lignée d'hybridomes DSM ACC2022.

2. Utilisation selon la revendication 1, caractérisée en ce que l'anticorps monoclonal est produit par la lignée d'hybridomes DSM ACC2022.

3. Utilisation d'un partenaire de liaison spécifique, qui est caractérisé en ce qu'il se fixe au même épitope que l'anticorps monoclonal qui est produit par la lignée d'hybridomes DSM ACC2022.

4. Utilisation de protéines de fusion ayant les propriétés de liaison à l'antigène d'un anticorps monoclonal selon la revendication 1.

5. Utilisation selon la revendication 1, caractérisée en ce que les segments ne se liant pas à l'antigène dans la séquence d'aminoacides des anticorps correspondent à une séquence humaine.

6. Utilisation d'une lignée d'hybridomes qui produit des anticorps monoclonaux selon la revendication 1.

7. Utilisation de la lignée d'hybridomes portant le numéro de dépôt DSM ACC2022.

8. Utilisation d'un acide nucléique ayant la séquence nucléotidique selon la figure la ou 1b.

9. Utilisation d'un polypeptide ayant la séquence d'aminoacides selon la figure 1a ou 1b.

10. Utilisation d'un anticorps selon la revendication 1, dans un agent de diagnostic in vitro.

11. Utilisation selon la revendication 10, pour la détection d'antigènes associés à des tumeurs dans des échantillons biologiques.

12. Utilisation d'un anticorps selon la revendication 1, pour la préparation d'un agent de diagnostic in vivo.

13. Utilisation d'un anticorps selon la revendication 1, pour la préparation d'un agent thérapeutique.
